# EUROPEAN PATENT APPLICATION

(11) **EP 2 169 643 A1**
(43) Date of publication of application: **31.03.2010**
(21) Application number: 09170534.3
(22) Date of filing: 17.09.2009
(51) Int. Cl.: G08B 21/04, G08B 21/12, A61B 5/00, H04M 1/725

(54) **Georeferenced mobile terminal for simultaneous measurement of environmental and biometric data in vivo having bidirectional radio connection with a processing centre**

(30) Priority: 19.09.2008 IT RM20080499
(71) Applicant: ENEA - Ente per le Nuove Tecnologie, l'Energia e l'Ambiente, 00196 Roma (IT); OCTO Telematics S.p.A., 42100 Reggio Emilia (RE) (IT)
(72) Inventor: Scafè, Raffaele, I-00061, Anguillara Sabazia (RM) (IT); Salmi, Maurizio, I-00198, Roma (RM) (IT); Cantoni, Mauro, I-00060, Riano (RM) (IT); Amendola, Roberto, I-00152, Roma (RM) (IT); Zuco, Giuseppe, I-00149, Roma (RM) (IT); Romanazzo, Maurizio, I-00069, Trevignano Romano (RM) (IT)
(74) Representative: Sarpi, Maurizio

(57) **Abstract**

A mobile device (TM) substantially comprises six functional units: a first unit (UGA), dedicated to the detection of environmental quantities; a second unit (UGF), dedicated to the detection of physiological and biometric quantities; a third unit (UG), dedicated to the georeferenced location of the device or terminal itself; a fourth unit (UCG) that is able to process the environmental, biometric and space-time data, as well as to transmit them to an operating centre and/or receive instructions therefrom; a fifth unit (UC) that is able to communicate in bi-directional mode with the data-processing centre; and a sixth unit (UA) that provides adequate electric-power supply for the circuits.

## Description

The present invention relates to the sector of devices for monitoring and detection of environmental parameters and biometric data *in vivo.* More in particular, it concerns a mobile terminal equipped with bidirectional radio connection with a mobile-terminal processing centre, which enables, in association with a plurality of mobile devices of a similar type, creation of a network for measuring environmental and biometric quantities operating within a preselected geographical area, which is explored dynamically by a set of georeferenced terminals connected in real time with a data-processing centre, the modality of exploration being established by the nature of the carriers that house the terminals themselves.

Amongst environmental parameters (the examples do not exhaust the range of the possible embodiments), the terminal is able to detect values of climatic parameters, of concentrations of dust suspended in the air, of doses of ionizing and non-ionizing radiation, etc., whilst amongst physiological parameters of the organism associated to the terminal itself, the latter is able to measure *in vivo* values of content of oxygen in the blood, of heart rate, of alcohol content in the blood, etc.

The interpretation of the set of the data obtained from a particular mobile terminal, together with possible other data available for the specific geographical area considered by other similar terminals or by some other source, enables, for example, identification of anomalous situations and formulation of appropriate preventive countermeasures for the reduction of potential imminent risks.

Devices for monitoring ionizing and non-ionizing radiation have been used for some time in nuclear power stations, in stations for outdoor measurements located throughout the territory and in laboratories that use radioactive material, according to the standards corresponding to safety and safeguarding of the environment. However, said devices are not mobile and enable measurement and control of the level of one or more types of radiation only in the area in which they are located. Said devices, moreover, are not able to carry out the measurement and detection of biometric parameters *in vivo.*

Still remaining within the case of radiometric sensors, with the greater attention that environmental themes call for, there is sharply felt the need to extend the measurements of fields of radiation to more extensive territories in order to draw up radiometric maps at a provincial, regional, or national level, as likewise to specific areas such as volcanic ones, without resorting to the location of an excessive number of fixed detection stations.

It would moreover be necessary for a system for monitoring the territory to enable an operating centre to evaluate in real time the evolution of a critical local situation that could extend into the surrounding territories, where the fixed stations of detectors may even be absent, supplying data coming from detectors installed on vehicles already autonomously in movement on the territory itself. Consider, for example, the rapid displacement of a radioactive cloud and the need for the Civil Protection to issue an alert in real time to the population that could come into contact with said cloud.

From the patent application No. USA 2007205891A1 there has recently been made known a monitoring system based upon a network of radiation detectors. Said system, however, albeit envisaging for each detector a plurality of sensors of a type different from one another associated for measurement of the different types of radiation, does not include any device for detection of the position of the detector itself and hence regards a network of sensors fixed with respect to the operating theatre, which cannot be identified and localized during and following upon a possible displacement thereof in so far as their mobile application is not envisaged.

The task of the present invention is to overcome the limits of the devices and of the systems so far known, by proposing a monitoring system based upon a network of bio-environmental terminals that will be able to move with respect to the operating theatre and that will be spatially locatable.

The above has been obtained according to the invention by providing a mobile bio-environmental terminal characterized by: (a) the capacity for making simultaneous measurements of environmental and biometric interest; (b) an even minimal degree of mobility on the theatre of interest in the course of the working life of the terminal itself; (c) the capacity to determine the space-time co-ordinates associated to the terminal itself referred to a reference system, which is fixed with respect to the Earth and has a time reference; (d) the capacity to communicate the data acquired to a data-processing centre, dedicated for said purpose, storing them in the case where, owing to lack of field or for different reasons of expedience it is not possible or not desirable to send them to said centre; (e) the capacity for receiving processed information, updatings and instructions by the data-processing centre itself.

By integrating said characteristics, the terminals forming the subject of the present invention are able to supply bio-environmental data describing the situation that interacts with the sensors throughout the path followed by the terminal itself during the acquisition time.

Obviously, the terminal will be able to supply bio-environmental data not only when it is moving but also when it is stationary.

According to the invention, the mobility of the terminal can derive from: (A) the installation on board a generic carrier of any type whatsoever, driven by a human being or else automatically, whether motor-powered or not; (B) the movement of the person or animal that is wearing it.

A peculiar characteristic of the invention is represented by the fact that the detectors are georeferenced via a locating system.

The environmental and biometric measuring devices, which may be housed on board a mobile terminal, must in principle respond not only to *automotive* specifications but also to requisites of compactness, capacity of continuous operation without interventions for replacement of parts (e.g., filters, reagents, etc.) for a period of time in the region of a few years and, finally, capacity for supplying data in digital form compatible with telematic processing. On this basis, it is possible to select quite an extensive range of commercial sensors both in the environmental field and in the biometric field.

By way of example, the following may be cited:
- amongst sensors of an environmental type:
   - meters for measuring concentration of fine dust in the air;
   - temperature and humidity sensors;
   - dosimeters of ionizing radiation;
   - meters for measuring the electromagnetic field;
   - meters for measuring concentrations of gaseous pollutants;
   - UV dosimeters;
   - dosimeters for sound waves;
   - etc.
- amongst sensors of a biometric type:
   - sensors for detecting fingerprints;
   - sensors for detecting the iris;
   - sensors for detecting oxygen content in the blood via pulsed light;
   - sensors of glycaemic content in the blood;
   - sensors of alcohol content in the blood;
   - sensors of alcohol content in the sweat;
   - alkaloid sensors (at present within solutions);
   - etc.

Basically, the present invention enables remote monitoring of values of environmental and biometric parameters even over geographical areas however extensive and in multiple operating conditions (e.g., underground environments, mines, submarine environments, high-altitude environments, etc.).

In particular, the invention renders possible a continuous and distributed recording on the territory of the levels of concentration of certain pollutants and of the values of temperature and humidity, the former being useful for integrating the measurements made by the fixed centres and the latter being useful for improving (after prior adequate filtering in the data-processing stage) the forecasting aspects of the meteorological models.

In the field of safety in transportation, the invention enables association to the monitoring of the style of driving of the means of transport (through an analysis of the evolution of the kinematic parameters or in perspective through connection to the can bus of the control unit of the vehicle), monitoring of critical microclimatic conditions within the passenger compartment and of physiological parameters through a purposely provided sensorized bracelet (heart beats, content of alcohol, level of oxygen in the blood, alkaloids, etc.) for determination of states at risk, such as: inebriation, seizures, conditions of drowsiness (evolution of the correlation between oxygen content and heart beats).

In the field of basic studies of the risk from radiation, the present invention makes available - unlike what has so far been possible - a plurality of environmental and *in vivo* physiological data acquired simultaneously and in a way associated to the space-time reference. Said set of data provides decisive elements for studies of the correlations between environmental and physiological conditions on the occasion of particular events. For example, said data are of considerable importance both for the study of the effects of balancing between the action of damage from radiation and the tissue-mediated mechanisms of repair, and for the damage to DNA produced by the interaction of the radiation and the physiological mechanisms of cell recovery. Studies in this sense have been underway for several decades on ionizing radiation, above all in the so-called "low-dose" domain.

In the field of physical surveillance of workers exposed to risk, the invention makes possible control of the individual exposure to ionizing and/or non-ionizing radiation simultaneously with the values of some physiological variables in order to evaluate the individual susceptibility to exposure to radiation and to determine possible early physiological effects thereof. Said checks, envisaged by the State by standards for the protection of health in workplaces and elsewhere, are carried out ex *post factum* by means of distinct (ionizing and non-ionizing) dosimetric systems operating in off-line mode whilst the clinical checks on the subjects exposed, where envisaged, are normally deferred in time.

In this sense the invention, in connection with the processing centre and possibly in association with a plurality of similar devices located over the territory, supplies the operating base for those structures and organizations that, for juridical reasons or else for compulsory application of standards, require said data. Amongst the former there may be cited: departments in operating areas with radiological risk, departments with tasks of civil protection, etc.

Considering the characteristics of the mobile terminals that are described and the mobility of the carriers by which they are conveyed into areas of interest, it is possible to identify merely by way of non-exhaustive example certain advantageous fields of application concerning the safety of the transport of goods and persons and environmental monitoring, based precisely upon the knowledge of the environmental and biometric data.

The invention enables remote monitoring of values of environmental and biometric parameters also in geographical areas, however extensive they may be, as well as in multiple operating conditions (e.g., underground environments, mines, submarine environments, high-altitude environments, etc.).

Further characteristics and advantages of the invention will emerge clearly from the ensuing detailed description with reference to the attached plates of drawings, which illustrate purely by way of nonlimiting example a preferred embodiment thereof.

In the drawings:

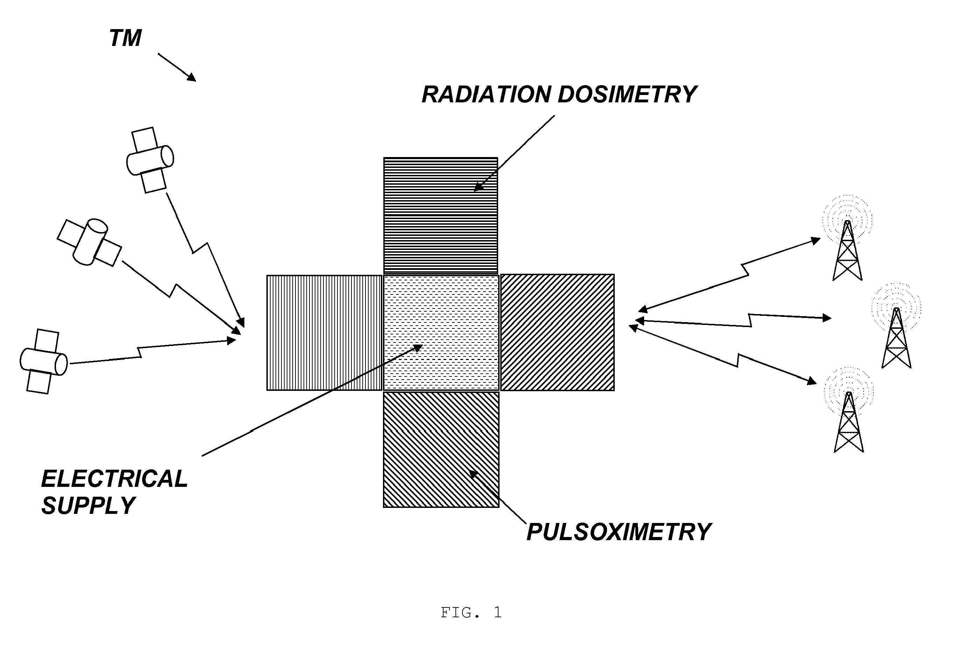

Figure 1 shows a functional diagram of the mobile terminal according to the invention; and

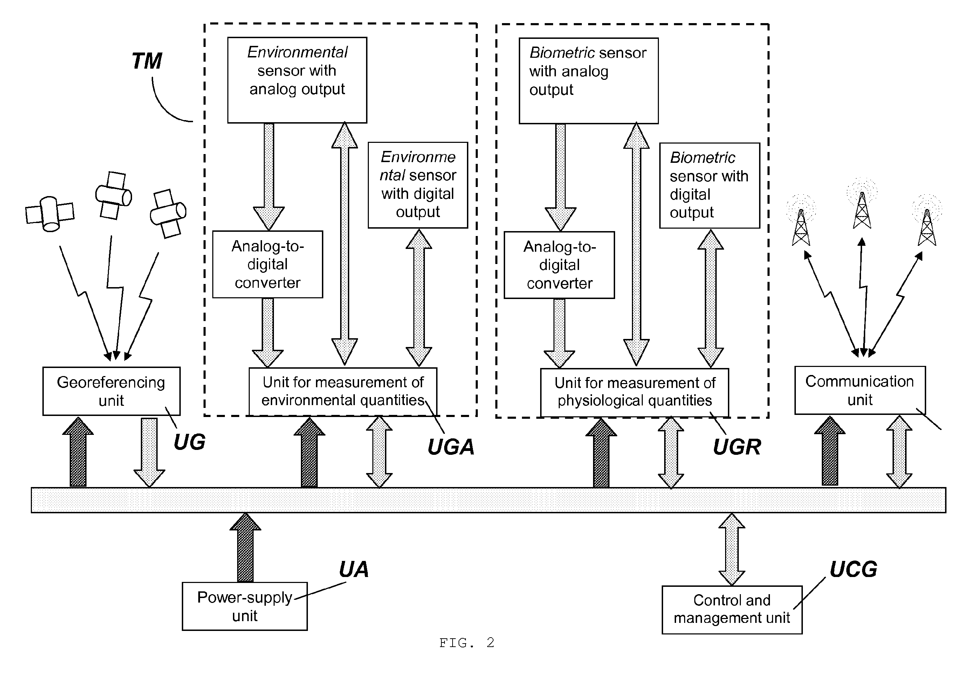

Figure 2 is a schematic illustration of a block diagram of the invention.

With reference to the figures, the device or mobile terminal, designated as a whole by TM, substantially comprises six functional units:
1. a unit UGA, dedicated to measurement of environmental quantities;
2.a unit UGF, dedicated to the measurement of physiological or biometric quantities;
3.a unit UG reserved to the georeferenced (satellite, earth or else hybrid) location of the terminal itself;
4. a management and control unit UCG, which is able to process the environmental, biometric, and positional data, as well as to regulate the parameters of operation and measurement, and control the functions of the entire apparatus, also according to inputs received from an operating centre;
5. a communication unit UC, designed to establish a bi-directional connection with the data-processing centre for sending/exchanging the processed data; and
6.a unit UA, which provides adequate electric-power supply for the circuits.

The individual elements or functional units making up the device TM may obviously be of different types and present different characteristics according to the specific requirements of measurement envisaged for the terminal, according to the technologies of location available and according to the modes of remote communication available and practicable in the territory of interest. Finally, corresponding to the temporal operating mode envisaged is the choice of the appropriate electrical supply.

In particular, of the above cited blocks of the device:
the unit UGA for measuring environmental quantities is equipped with environmental sensors of an analog and digital type, with corresponding A/D converter, comprising: meters for measuring concentration of fine dust in the air, temperature and humidity sensors, dosimeters of ionizing radiation, meters for measuring the electromagnetic field, meters for measuring concentrations of gaseous pollutants, UV dosimeters, dosimeters for sound waves, etc.
the unit UGF for measuring physiological and biometric quantities is equipped with biometric sensors of an analog and digital type, with corresponding A/D converter, comprising: sensors for detecting fingerprints, sensors for detecting the iris, sensors for detecting oxygen content in the blood via pulsed light, sensors for detecting glycaemic content in the blood, sensors for detecting of alcohol content in the blood, sensors for detecting of alcohol content in the sweat, alkaloid sensors (at present within solutions), etc.
the unit for georeferencing UG and for location of the device TM comprises - by way of non-exhaustive example of the satellite methodology - Global Navigation Satellite Systems (GNSS) receivers that make available the signals indispensable for associating the necessary absolute references of a temporal and spatial type to the environmental and biometric measurements.

The communication unit UC with the remote processing centre operates in bi-directional mode with a radio communication system based, by way of non-exhaustive example of the possibilities, upon GSM, GPRS, or UMTS technology. The results of the measurements are appropriately returned in digital form, biuniquely associated to the sensors installed and georeferenced before being transmitted in selective or cumulative mode to at least one remote receiving station, not represented in the figures, via a communication network of a known type.

The control and management unit UCG of the invention, in addition to carrying out management of the parameters of operation and measurement and control of the functions of the entire apparatus, comprises means for processing the data to be sent to/exchanged with the remote processing centre, as well as means for nonvolatile storage of the aforesaid data to enable postponement of sending thereof to the receiving station either by choice or out of necessity.

Finally, the unit UA for electrical supply of the circuits must respond, in addition to the normal criteria of sizing, also to the particular uses that are required of the terminals TM. By this it is intended to specify and comprise, by way of non-exhaustive example of the cases:
(a) the devices supplied by sources with which the carriers that house them are equipped;
(b) the devices supplied by sources recharged via the ones with which the carriers that house them are equipped;
(c) the devices supplied by sources that can run down, which enable operation for a limited time (disposable devices); and
(d) the devices supplied by rechargeable sources through external sources and available in the operating environment.
Amongst the advantages of the device of the present invention the following should be listed:
- the terminals TM do not require local intervention of operators in so far as they are autonomous and controlled in a remote way by a purposely provided data-processing centre;
- a practically unlimited number of terminals TM can be added to or removed from the network, which can thus increase or decrease in a dynamic way in time;
- the type of the measurements made of the terminals TM is not fixed *a priori* and this enables connection of the terminals to sensors of any type;
- the means equipped with terminals TM are not forced to follow obligate paths or else make purposely planned stops that can in practice lead to a reduction in the intensity of the environmental and biometric checks to facilitate mobility of the goods and/or persons; the measurements are in fact made during the journey;
- possible situations of alert can be effectively managed, there being available in real time, not only the environmental and biometric data, but also the data of spatial and temporal tracking of the terminal TM;
- it is possible to draw up and manage a mapping in real time of the territory, also for purely statistical reasons (and not only in the case of emergency), accumulating the measurements made by the plurality of the devices so as construct a historical file.

A preferred embodiment of the invention has been described so far herein. It is on the other hand evident that numerous variations and modifications may be made by a person skilled in the branch to the device described in order to adapt it to the different operating requirements, without on the other hand departing from the sphere of protection of the present industrial patent right, as defined by the ensuing claims.

## Claims

1. A mobile device or terminal (TM) for detecting environmental and biometric parameters, **characterized in that** it is designed to be transported by a mobile vector and **in that** it comprises, in combination:
a) means designed to make measurements of an environmental type;
b) means designed to make measurements of a biometric type;
c) means for determining the space-time co-ordinates associated to the device itself referred to a reference system, which is fixed with respect to the Earth and is provided with a time reference, or else to another location system; and
d) means for communicating the environmental and biometric data acquired and the corresponding space and time references to a data-processing centre dedicated to said purpose;
thus obtaining that said device has a degree of mobility, even minimal, in the context of interest in the course of the working life of the device itself and is able to supply to said processing centre environmental and biometric data detected in all the geographical positions occupied thereby during the acquisition time.

2. The device as per the preceding claim, **characterized in that** it substantially comprises six functional units:
- a first unit (UGA) dedicated to detection of environmental quantities;
- a second unit (UGF) dedicated to detection of physiological and biometric quantities, as well as to regulation of the parameters of operation and measurement and to control of the functions of the entire apparatus, also according to inputs received from an operating centre;
- a third unit (UG) dedicated to the georeferenced location of the device or terminal itself;
- a fourth unit (UCG) that is able to process the environmental, biometric, and space-time data;
- a fifth unit (UC) that is able to communicate in bi-directional mode with the data-processing operating centre; and
- a sixth unit (UA) that provides the adequate electric-power supply for the circuits.

3. The device as per Claim 2, **characterized in that** the first unit (UGA) for measurement of environmental quantities is equipped with environmental sensors of an analog and/or digital type, with corresponding A/D converter, comprising: meters for measuring concentration of fine dust in air, temperature and humidity sensors, dosimeters of ionizing radiation, meters for measuring the electromagnetic field, meters for measuring concentrations of gaseous pollutants, UV dosimeters, dosimeters for sound waves, etc.

4. The device as per Claim 2, **characterized in that** the second unit (UGF) for measurement of physiological and biometric quantities is equipped with biometric sensors of an analog and/or digital type, with corresponding A/D converter, comprising: sensors for detecting fingerprints, sensors for detecting the iris, sensors for detecting oxygen content in the blood via pulsed light, sensors for detecting blood glucose concentration, sensors for detecting alcohol content in the blood, sensors for detecting alcohol content in the sweat, sensors for detecting alkaloids (at present into solutions), etc.

5. The device as per Claim 2, **characterized in that** the third unit (UG) for georeferencing and location of the device (TM) comprises Global Navigation Satellite System (GNSS) receivers that make available the signals indispensible for associating to the environmental and biometric measurements the necessary absolute references of a time and space type.

6. The device as per Claim 2, **characterized in that** the fourth unit (UCG) for control and management of the terminal (TM) comprises means for processing the data to be sent to/exchanged with the remote processing centre, as well as means for nonvolatile storage of the aforesaid data to enable postponement of sending thereof to the receiving station by choice or by necessity.

7. The device as per Claim 2, **characterized in that** the fifth unit (UC) for communication with the remote processing centre is designed to operate in bi-directional mode with a radio communication system based upon GSM and/or GPRS and/or UMTS technology; the results of the measurements being such that they can be appropriately returned in digital form, biuniquely associated to the sensors installed and georeferenced before being transmitted in selective or cumulative mode to at least one remote receiving station via a communication network of a known type.

8. The device as per Claim 2, **characterized in that** the sixth unit (UA) for electrical supply of the circuits is designed to operate with:
energy sources with which the carriers that house the terminal (TM) itself are equipped;
sources recharged via the ones with which the carriers that house the terminal (TM) itself are equipped;
sources that can run down, which enable operation for a limited time (disposable devices); and
sources rechargeable through external sources available in the operating environment.

9. The device as per Claim 1, **characterized in that** it moreover comprises means of a known type for storing the environmental and biometric data acquired and the corresponding space and time references so as to be able to transmit them to the data-processing centre in a deferred way.

10. The device as per any one of the preceding claims, **characterized in that**, in association with a plurality of mobile devices (TM) of a similar type, it is designed to enable provision of a network for measuring geographically distributed environmental and biometric quantities, which operates in real time within a pre-selected geographical area which is explored dynamically by at least one of said georeferenced devices.

11. A network for measuring geographically distributed environmental and biometric quantities comprising:
a plurality of mobile devices (TM) according to Claim 1 or Claim 9, which explore a pre-selected geographical area dynamically; and
a remote processing centre, which is configured for communicating with said mobile devices (TM) and for receiving, also in real time, the environmental and biometric data that interact with each of said mobile devices (TM) in all the geographical positions occupied by the devices themselves during the acquisition time.
